# EUROPÄISCHE PATENTSCHRIFT

(11) **EP 1 985 262 B1**
(45) Veröffentlichungstag und Bekanntmachung des Hinweises auf die Patenterteilung: **24.04.2013**
(21) Anmeldenummer: 08006201.1
(22) Anmeldetag: 29.03.2008
(51) Int. Cl.: A61F 2/90

(54) **Stent**
Stent
Stent

(30) Priorität: 26.04.2007 DE 102007019703
(43) Veröffentlichungstag der Anmeldung: 29.10.2008
(73) Patentinhaber: Biotronik VI Patent AG, 6341 Baar (CH)
(72) Erfinder: Lootz, Daniel, 18119 Rostock (DE); Surber, Bettina, 8600 Dübendorf (CH); Haussmann, Mathias, 8005 Zürich (CH)
(74) Vertreter: Lindner-Vogt, Karin L.

(56) Entgegenhaltungen:
- WO-A1-2006/020649
- WO-A2-02/065949
- WO-A2-2006/116383
- WO-A2-2006/138010

## Beschreibung

Die Erfindung betrifft einen Stent mit den im Oberbegriff des Patentanspruches 1 angegebenen Merkmalen.

Ein derartiger Stent ist beispielsweise aus der US 2006/0122694 A1 bekannt und umfasst eine im Wesentlichen röhrenartige, offene Tragstruktur aus miteinander verbundenen Stegen. Diese Tragstruktur ist unter Deformation der aus zusammenhängenden Streben abschnittsweise gebildeten Stege zur Applikation des Stents in einem Gefäß des Patienten radial aufweitbar.

Bei dem vorbekannten Stent sind ferner in die Tragstruktur Sollbruchstellen integriert, die zur Fragmentierung des Stents nach der Applikation dienen. Wie in der angegebenen US-Offenlegungsschrift erörtert ist, dient diese Fragmentierung dazu, die von den umlaufenden Streben gebildete, elektrisch leitfähige Leiterschleife aufzubrechen, um so die Sichtbarkeit des Körpermaterials in dem vom Stent umgebenen Lumen in einer Magnetresonanzuntersuchung zu erhöhen. Da während der Applikation des Stents die Integrität der Stege noch gewährleistet ist, weist dieser Stent eine genügende Stabilität auf, um für die durch seine Applikation gewünschte Gefäßerweiterung Sorge zu tragen.

Bei dem bekannten Stent sind die Sollbruchstellen durch Materialbrücken innerhalb der Stentstege gebildet, die aus einem stärker korrosionsfähigen Material bestehen. Alternativ dazu können die Sollbruchstellen durch eine Querschnittsverengung gebildet sein, die aufgrund der stattfindenden Korrosion des Stents zu einer definierten Auflösung der Strukturstabilität des Stents im Bereich der Sollbruchstelle führt.

Aus der WO 2006/116383 A2 sind Stents bekannt, die Verbinder mit Sollbruchstellen aufweisen.

Die WO 2006/138010 A2 offenbart Implantate zur Anwendung am Herzen. Unter anderem ist ein Stent beschrieben, der an der Stelle der Verbinder erweiterte Regionen mit beispielsweise Bohrungen aufweist. Durch diese Bohrungen wird ein Verbindungsdraht gezogen, der die Regionen der Verbinder gelenkig zusammenhält [Fig. 19D].

Die jüngere Entwicklung auf dem Gebiet von Stents sieht als Material Magnesium und dessen Legierungen vor. Diese Materialien sind biodegradierbar und führen somit zu einer gewünschten Auflösung des Stents nach Aufweitung des Gefäßes und dessen Eigenstabilisierung. Damit wird das Problem verhindert, dass der als Fremdkörper wirkende Stent mit der Zeit zu Zellanlagerungen neigt und damit das therapierte Gefäß durch eine Restenose wieder verschlossen wird.

Magnesium und dessen Legierungen sind allerdings bei korrosiver und/oder gleichzeitig schwingender Beanspruchung, wie sie ein im pulsierenden Blutstrom applizierter Stent unterliegt, bruchgefährdet. Zusätzlich können örtliche Korrosionsstellen, beispielsweise aufgrund von Ungleichmäßigkeiten im Werkstoff oder der Oberfläche, auftreten. Stents, wie sie beispielsweise aus der EP 1 430 854 A1 bekannt sind, können damit nach der initialen Umformung zur radialen Aufweitung des Stents einer Gefahr der unkontrollierten Fragmentierung unterliegen, so dass die stützende Funktion eines solchen Stents, insbesondere in den arteriellen Blutgefäßen, gefährdet ist. Der Grund hierfür liegt darin, dass einzelne Stegelemente im Bereich einer unkontrollierten Fragmentierung dadurch keinen strukturmechanischen Zusammenhalt mehr aufweisen, mit zunehmender Anzahl der gebildeten Fragmente geht die stützende Funktion des Stents verloren.

Ausgehend von der geschilderten Problematik des Standes der Technik liegt der Erfindung die Aufgabe zugrunde, einen Stent der gattungsgemäßen Art so weiterzubilden, dass insbesondere bei Verwendung eines bruchgefährdetem Materials für den Stent eine unkontrollierte Fragmentierung vermieden wird.

Diese Aufgabe wird durch die im Kennzeichnungsteil des Anspruches 1 angegebenen Merkmale gelöst, wonach im Bereich der Sollbruchstellen des Stents eine Gelenkausbildung der an die jeweilige Sollbruchstelle angrenzenden Enden der die Stege bildenden Streben derart vorgesehen ist, dass die Enden der Streben bei gebrochener Sollbruchstelle unter Druckbeaufschlagung des Stents gelenkig zusammen gehalten sind.

Die Erfindung beruht also gegenüber dem Stand der Technik auf einem diametral entgegengesetztem Konzept. Es wird nämlich eine kontrollierte Fragmentierung im Bereich der Sollbruchstellen der Tragstruktur verfolgt, so dass der Zeitpunkt des lokalen Brechens von Stentbereichen definiert erfolgt und durch eine Auslegung des Stents in seiner Formgebung steuerbar gemacht wird. Durch die Gelenkausbildung im Bereich der Sollbruchstellen wird in diesem Zusammenhang eine Strukturschwächung so weit vermieden, dass der Stent trotz der Fragmentierung seine Stützwirkung noch eine genügend lange Zeit aufrechterhalten kann. Grund hierfür ist die kontrollierte Zusammenführung der an die Sollbruchstelle angrenzenden Enden der Streben durch die vom Gefäß herrührende radiale Belastung. Die Streben wirken also trotz Integrationsverlust unter Druck radial stützend.

Ein weiterer Vorteil dieser kontrollierten Fragmentierung liegt darin, dass der Stent damit in seinem Aufbau besser an Materialien mit einer reduzierten Bruchdehnung bzw. Zug- oder Biegefestigkeit anpassbar ist. Die kontrollierte Fragmentierung führt nämlich zu einem Spannungsabbau in den radial belastenden Bereichen, wodurch keine unkontrollierte Spannungskorrosion mehr stattfinden kann.

In den abhängigen Ansprüchen sind bevorzugte Ausführungsformen des erfindungsgemäßen Stents angegeben. Deren Merkmale, Einzelheiten und Vorteile werden zur Vermeidung von Wiederholungen anhand der nachfolgenden Beschreibung eines Ausführungsbeispiels des Erfindungsgegenstandes anhand der beigefügten Zeichnungen näher erörtert.

Es zeigen:
- Figur 1: eine Längsseitenansicht eines Stents,
- Figur 2: eine schematische ausschnittsweise Ansicht in Abwicklungsdarstellung von vier Stegreihen des Stents in kontrahiertem Zustand,
- Figuren 3 und 4: schematische Ansichten zweier benachbarten Strebenenden im Bereich einer Sollbruchstelle während und nach der Fragmentierung,
- Figuren 5: eine Ansicht der vier Stegreihen analog Fig. 2 in expandiertem Zustand des Stents,
- Figur 6: eine schematische, vergrößerte Ansicht der Enden zweier benachbarten Streben mit einer Sollbruchstelle in einer ersten alternativen Ausführungsform, und
- Figur 7: einen schematischen Radialschnitt durch einen Stent mit einer Sollbruchstelle in einer zweiten alternativen Ausführungsform.

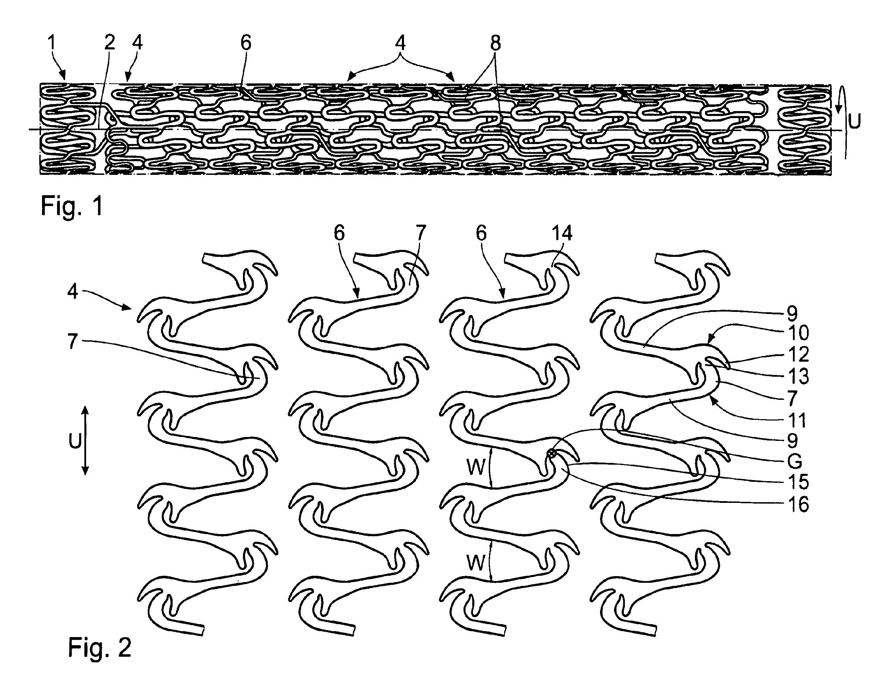

In Fig. 1 ist ein Stent 1 dargestellt, der eine zylindrische Grundform mit einer Längsachse 2 aufweist. Seine Umfangswandung ist aus einer röhrenartigen, offenen Tragstruktur 4 gebildet, die aus miteinander verbundenen Stegen 6 besteht. Diese Stege 6 weisen in Umfangsrichtung U eine helikale Primärform auf. Als diese überlagernde Sekundärform sind die Stege 6 in Umfangsrichtung mäanderförmig ausgebildet, wobei die Mäanderbögen abwechselnd zu den Stentenden hin weisende Zenite 7 aufweisen. An vereinzelten Positionen sind die nebeneinander verlaufenden Stege 6 durch Axialverbinder 8 miteinander verbunden. Das gesamte Gitterwerk der Tragstruktur 4 ist aus einem zylinderförmigen Rohling, beispielsweise aus einer Magnesiumlegierung, durch Laserschneiden herausgearbeitet.

In Fig. 2 ist die in Fig. 1 der Übersichtlichkeit halber weggelassene Feinstruktur der Stege 6 näher dargestellt. Die Mäanderform der Stege 6 ist abschnittsweise durch zick-zack-förmig hin- und hergehende Streben 9 zwischen den Zeniten 7 zusammengesetzt. Die Enden 10, 11 der Streben 9 sind jeweils unterschiedlich aufgebaut. Das eine Strebenende 10 ist jeweils zu einer gelenkpfannenartigen Formgebung 12 ausgeformt, während das jeweils gegenüberliegende Ende 11 als eine Gelenkkugel 13 ausgebildet ist. Die Gelenkkugel 13 ist dabei am Ende einer mit einer Außenrundung 15 versehenen Abkröpfung 16 gelegen. Der Durchmesser der Gelenkkugel 13 ist gegenüber dem Innendurchmesser der Gelenkpfanne 12 deutlich kleiner. Gelenkpfanne 12 und Gelenkkugel 13 sind - da die gesamte Struktur des Stents aus der Mantelfläche des erwähnten Rohlings ausgeschnitten ist - bezüglich der eigentlichen Gelenkstelle als zweidimensional zu bezeichnen, so dass ihre Gelenkachse G radial gerichtet ist.

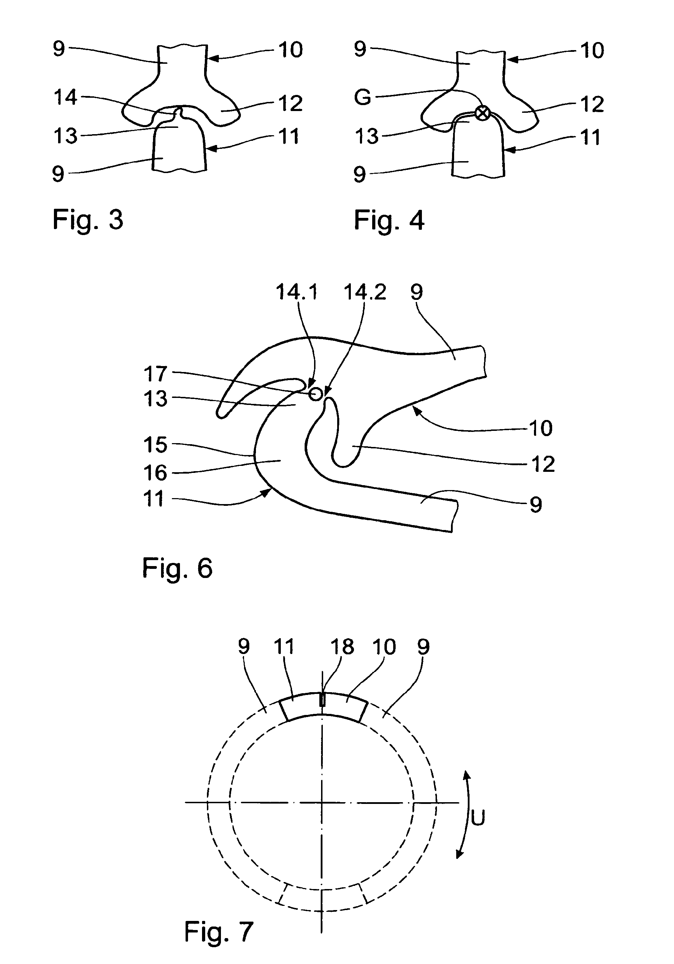

Wie aus Fig. 3 deutlich wird, ist zwischen der Gelenkpfanne 12 und der Gelenkkugel 13 ein einstückig ausgeformter Bruchsteg 14 vorgesehen, der innerhalb der Gelenkausbildung aus Gelenkpfanne 12 und Gelenkkugel 13 als Sollbruchstelle für die Tragstruktur 4 fungiert. Die schmalen Bruchstege 14 weisen eine gegenüber dem eigentlichen Steg um ein Vielfaches geringere Querschnittsfläche durch ihre geringe Breite auf.

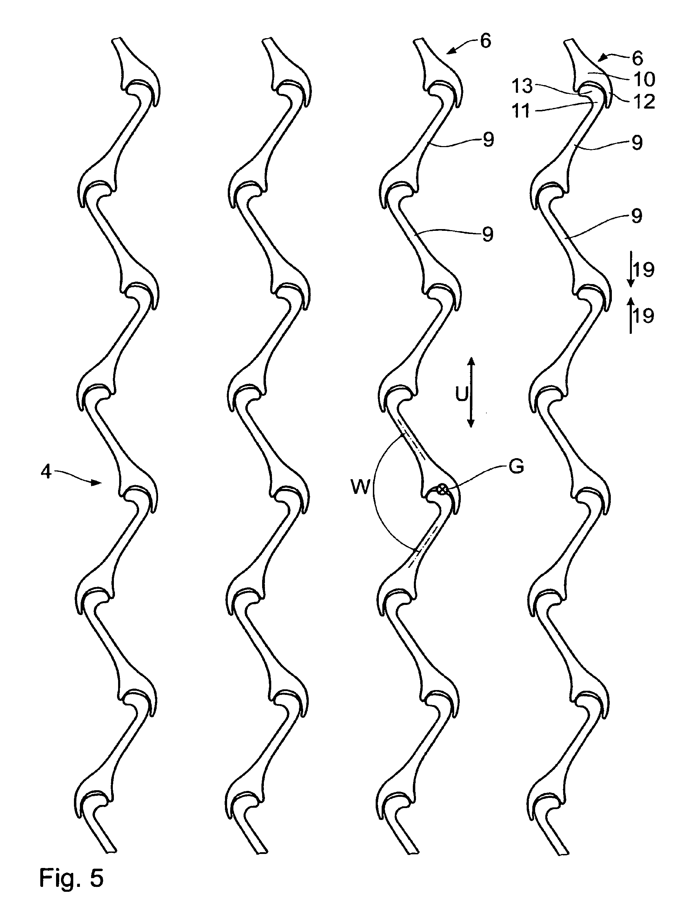

Wie aus den Fig. 2 bis 5 deutlich wird, werden beim Expandieren des Stents 1 während seiner Applikation in einem Gefäß die Winkel W zwischen den Streben 9 durch Aufbiegen extrem vergrößert. Das auf die Streben ausgeübte Biegemoment sorgt dabei für ein Brechen der Bruchstege 14, die Sollbruchstellen innerhalb der Stege 6 werden also ausgelöst. Unter dem radialen Druck, der von dem Gefäß auf den Stent 1 ausgeübt wird, kommen die Gelenkkugeln 13 sauber in den entsprechenden Gelenkpfannen 12 zu liegen und werden so gelenkig zusammengehalten. Dies bedeutet, dass die Aufweitung des Stents ohne Beaufschlagung der Stentstruktur mit problematischen Spannungsspitzen erfolgen kann. Die die Gelenkpfanne 12 und Gelenkkugel 13 zusammenhaltenden Kräfte, die durch die radiale Beaufschlagung des Stents durch das Gefäß erzeugt wird, sind in Fig. 5 durch den Pfeil 19 angedeutet.

Bei der in Fig. 6 gezeigten Ausführungsform ist die Sollbruchstelle zwischen den beiden Enden 10, 11 zweier benachbarten Streben 9 durch ein radial in die Tragstruktur eingebrachtes Durchgangsloch 17 ausgebildet, so dass zwei nebeneinander verlaufende Bruchstege 14.1, 14.2 vorliegen. Alternativ dazu kann - wie Fig. 6 zeigt - eine Sollbruchstelle zwischen zwei benachbarten Enden 10, 11 der Streben 9 durch ein Sackloch oder -schlitz 18 vorgesehen werden.

Generell kann eine Querschnittsreduktion auf bis zu 10% der Querschnittsfläche der Streben 9 vorgenommen werden. Dies richtet sich nach dem jeweiligen Material und kann durch einfache Versuche ermittelt werden.

## Patentansprüche

1. Stent umfassend
- eine Umfangswandung aus einer im Wesentlichen röhrenartigen, offenen Tragstruktur (4), die aus miteinander verbundenen Stegen (6) besteht, wobei die Stege (6) mäanderförmig in Umfangsrichtung ausgebildet sind, wobei die Mäanderbögen der Stege (6) abwechselnd zu den Stentenden hinweisende Zenite (7) aufweisen und wobei die Mäanderbögen der Stege (6) abschnittsweise gebildet sind durch zick-zack-förmig hin- und hergehende Streben (9) zwischen den Zeniten (7), wobei die Tragstruktur (4) unter Deformation der Streben (9) zur Applikation des Stents radial aufweitbar ist, und
- Sollbruchstellen (14) in der Tragstruktur (4) zur Fragmentierung des Stents nach der Applikation,
**gekennzeichnet durch**
- eine im Bereich der Sollbruchstellen (14) vorgesehene Gelenkausbildung (12, 13) der an die jeweilige Sollbruchstelle (14) angrenzenden Enden (10, 11) der Streben (9) derart, dass die Enden (10, 11) der Streben (9) bei gebrochener Sollbruchstelle (14) unter Druckbeaufschlagung des Stents gelenkig zusammengehalten werden.

2. Stent nach Anspruch 1, **dadurch gekennzeichnet, dass** die Enden (10, 11) der an eine jeweilige Sollbruchstelle (14) angrenzenden Streben (9) als Pfannengelenk mit einer gelenkpfannenartigen Formgebung (12) am einen Strebenende (10, 11) und einer gelenkkugelartigen Formgebung (13) am anderen Strebenende (10, 11) ausgebildet sind.

3. Stent nach Anspruch 2, **dadurch gekennzeichnet, dass** das Pfannengelenk eine radial bezüglich des Stents verlaufende Gelenkachse (G) aufweist.

4. Stent nach einem der vorgenannten Ansprüche, **dadurch gekennzeichnet, dass** die Sollbruchstellen als die Enden (10, 11) der jeweiligen Streben (9) verbindende Bruchstege (14) ausgebildet sind, die eine gegenüber den Streben (9) geringere Querschnittsfläche aufweisen.

5. Stent nach Anspruch 4, **dadurch gekennzeichnet, dass** die Bruchstege (14) gegenüber den Streben (9) eine geringere Breite aufweisen.

6. Stent nach Anspruch 4, **dadurch gekennzeichnet, dass** die Bruchstege (14) gegenüber den Streben (9) eine geringere Dicke aufweisen.

7. Stent nach Anspruch 4, **dadurch gekennzeichnet, dass** in einer Sollbruchstelle mindestens zwei Bruchstege (14.1, 14.2) vorgesehen sind.

8. Stent nach einem der vorgenannten Ansprüche, umfassend in Peripherrichtung mäanderförmig umlaufende Stege (6), **dadurch gekennzeichnet, dass** die Sollbruchstellen (14) mit der Gelenkausbildung im Bereich der Zenite (7) der Stege (6) angeordnet sind.

9. Stent nach Anspruch 2 und 8, **dadurch gekennzeichnet, dass** die gelenkkugelartige Formgebung (13) an einem Strebenende (11) im Bereich einer Sollbruchstelle (14) durch eine außen gerundete Abkröpfung (16) des Strebenendes (11) gebildet ist.

## Claims

1. A stent, comprising
- a peripheral wall made of a substantially tubular, open supporting structure (4), which consists of interconnected webs (6), wherein the webs (6) are formed in a meander-like manner in the peripheral direction, wherein the meander curves of the webs (6) have zeniths (7) pointing alternately toward the stent ends, and wherein the meander curves of the webs (6) are formed over portions by zigzag-shaped reciprocating struts (9) between the zeniths (7), wherein the supporting structure (4) can be radially expanded with deformation of the struts (9) for application of the stent, and
- predetermined breaking points (14) in the supporting structure (4) for fragmentation of the stent after application,
**characterised by**
- a joint formation (12, 13) of the ends (10, 11) of the struts (9) adjoining the respective predetermined breaking points (14), said joint formation being provided in the region of the predetermined breaking points (14) in such a way that the ends (10, 11) of the struts (9) are held together in an articulated manner in the event of a broken predetermined breaking point (14) under pressurisation of the stent.

2. The stent according to Claim 1, **characterised in that** the ends (10, 11) of the struts (9) adjoining a respective predetermined breaking point (14) are formed as a ball-and-socket joint with a socket-like shaping (12) at one strut end (10, 11) and a ball-like shaping (13) at the other strut end (10, 11).

3. The stent according to Claim 2, **characterised in that** the ball-and-socket joint has a joint axis (G) running radially with respect to the stent.

4. The stent according to one of the preceding claims, **characterised in that** the predetermined breaking points are formed as breaking webs (14) connecting the ends (10, 11) of the respective struts (9) and have a smaller cross-sectional area compared to the struts (9).

5. The stent according to Claim 4, **characterised in that** the breaking webs (14) have a smaller width compared to the struts (9).

6. The stent according to Claim 4, **characterised in that** the breaking webs (14) are thinner compared to the struts (9).

7. The stent according to Claim 4, **characterised in that** at least two breaking webs (14.1, 14.2) are provided in a predetermined breaking point.

8. The stent according to one of the preceding claims, comprising webs (6) running in a meandering manner in the peripheral direction, **characterised in that** the predetermined breaking points (14) with the joint formation are arranged in the region of the zeniths (7) of the webs (6).

9. The stent according to Claim 2 and 8, **characterised in that** the ball-like shaping (13) is formed at one strut end (11) in the region of a predetermined breaking point (14) by an externally rounded portion (16) of the strut end (11).

## Revendications

1. Stent comprenant
- une paroi périphérique constituée d'une structure portante ouverte (4), essentiellement du genre tuyau, réalisée avec des branches (6) reliées entre elles, dans lequel les branches (6) sont conçues en forme de méandres dans la direction du pourtour, dans lequel les courbes de méandres des branches (6) comportent des sommets (7) orientés en alternance vers les extrémités du stent et dans lequel les courbes de méandres des branches (6) sont partiellement formées par des entretoises (9) en forme de zigzag partant dans un sens et l'autre entre les sommets (7), dans lequel la structure portante (4) peut être élargie radialement par déformation des entretoises (9), pour l'application du stent, et
- des zones destinées à la rupture (14) dans la structure portante (4), pour la fragmentation du stent suite à l'application,
**caractérisé en ce que**
- une formation en articulation (12, 13) des extrémités (10, 11) d'entretoises (9) adjacentes à chacune des zones destinées à la rupture (14) est prévue dans la région des zones destinées à la rupture (14), de sorte que les extrémités (10, 11) des entretoises (9) sont maintenues ensemble de façon articulée, lors de la rupture de la zone destinée à la rupture (14) en soumettant le stent à une pression.

2. Stent selon la revendication 1, **caractérisé en ce que** les extrémités (10, 11) des entretoises (9) adjacentes à chacune des zones destinées à la rupture (14) sont conçues comme des joints à rotule, avec une formation du genre coussinet à rotule (12) à l'une des extrémités d'entretoise (10, 11) et une formation du genre sphérique (13) à l'autre extrémité d'entretoise (10, 11).

3. Stent selon la revendication 2, **caractérisé en ce que** le joint à rotule comporte un axe de joint s'étendant radialement par rapport au stent.

4. Stent selon l'une des revendications précédentes, **caractérisé en ce que** les zones destinées à la rupture sont conçues comme des branches de rupture (14) reliant les extrémités (10, 11) de chacune des entretoises (9), comportant une aire de section inférieure à celle des entretoises (9).

5. Stent selon la revendication 4, **caractérisé en ce que** les branches de rupture (14) présentent une largeur inférieure à celle des entretoises (9).

6. Stent selon la revendication 4, **caractérisé en ce que** les branches de rupture (14) présentent une épaisseur inférieure à celle des entretoises (9).

7. Stent selon la revendication 4, **caractérisé en ce qu'**au moins deux branches de rupture (14.1, 14.2) sont prévues dans une zone destinées à la rupture.

8. Stent selon l'une des revendications précédentes, comprenant des branches (6) s'étendant en forme de méandres dans la direction périphérique, **caractérisé en ce que** les zones destinées à la rupture (14) avec la formation en articulation sont agencées dans la région des sommets (7) des branches (6).

9. Stent selon les revendications 2 et 8, **caractérisé en ce que** la formation du genre sphérique (13) à une extrémité d'entretoise (11) dans la région d'une zone destinée à la rupture (14) est formée par un coude arrondi à l'extérieur (16) de l'extrémité d'entretoise (11).
